# EUROPEAN PATENT APPLICATION

(11) **EP 0 891 766 A1**
(43) Date of publication of application: **20.01.1999**
(21) Application number: 98112954.7
(22) Date of filing: 13.07.1998
(51) Int. Cl.: A61K 7/42

(54) **Light-screening agents**

(30) Priority: 14.07.1997 EP 97111938
(71) Applicant: F. HOFFMANN-LA ROCHE AG, 4070 Basel (CH)
(72) Inventor: Bringhen, Alain, 1257 Croix-de-Rozon (CH); Gonzenbach, Hans Ulrich, 1202 Geneva (CH); Pochon, Magali, 1213 Petit-Lancy, Geneva (CH)
(74) Representative: Kellenberger, Marcus, Dr.

(57) **Abstract**

The invention relates to a photostabilized dibenzoylmethane type UV-A screening agent stabilized by at least one compound of formula I 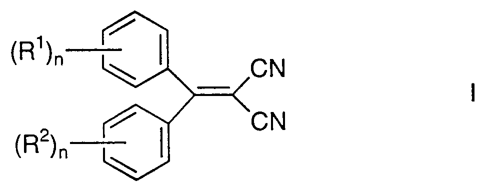
wherein R¹ and R² are equal or different and represent linear or branched alkyl or alkoxy radicals with 1 to 18 C atoms or one of R¹ and R² is a hydrogen atom and n is 1 or 2.

## Description

The invention relates to photostabilized light screening agents of dibenzoylmethane type UV-A, to cosmetic light-screening compositions containing at least one of the above agents, the use of the cosmetic light-screening compositions for the protection of the human skin and human hair against the ultraviolet radiation of wavelengths between 320 and 400 nm, and between 290 and 400 nm, respectively, and the use of a compound known per se as a stabilizer for dibenzoylmethane type UV-A agents.

It is known how to photostabilize a UV-A screening agent of the dibenzoylmethane type. Photostabilization in this context means to maintain a constant or nearly constant protection of human skin or hair by an UV-A screening agent against ultraviolet light in the range of 320 to 400 nm.

Photostabilization of an UV-A screening agent up to now is performed by adding to the UV-A agent at least one of the specific UV-B filter compounds known for this purpose. For example the stabilization of the UV-A screening agent 4-tert.butyl-4'-methoxydibenzoylmethane as e.g. disclosed in US patent No. 4,387,089 (which is sold as PARSOL 1789® by F.Hoffmann-La Roche AG) is performed by Octocrylene (see EP-A-780 119), benzylidenes (see EP-A-754445), especially by methylbenzyliden camphor, or by a polymer of the benzylidene malonate silicone type (see EP-A-708080), which photostabilizing compounds are all UV-B filters.

Surprisingly it has now been found that UV-A filter compounds of formula I 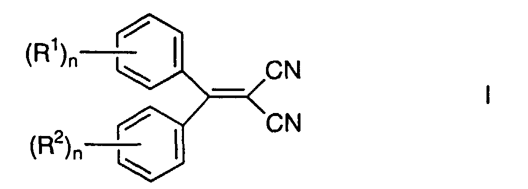
wherein R¹ and R² are equal or different and represent linear or branched alkyl or alkoxy radicals with 1 to 18 C atoms or one of R¹ and R² is a hydrogen atom and n can be 1 or 2,
photostabilize dibenzoylmethane type UV-A screening agents. In this respect compounds of formula I wherein R¹ and R² are alkoxy radicals, especially branched alkoxy radicals, with 3 to 12 C atoms or one of R¹ and R² is a hydrogen atom whereby R¹ and/or R² are/is in para-configuration and n is 1 and whereby an alkoxy radical out of the group consisting of n-propoxy-, isopropoxy-, n-butoxy-, 1-methylpropoxy-, 2-methylpropoxy-, n-pentoxy-, 1,1-dimethylpropoxy-, 3-methylbutoxy-, hexyoxy-, 2,2-dimethylpropoxy-, heptoxy-, 1-methyl-1-ethylpropoxy-, 2-ethylhexoxy- and/or octoxy- is of specific interest.

A preferred compound of formula I for stabilization of the dibenzoylmethane type UV-A screening agents is the compound of formula I wherein R¹ is an hydrogen atom and R² is H₉C₄O-.

Other suitable compounds of this particular type are: compound of formula I wherein R¹ and R² are both 2-ethylhexoxy and compound of formula I wherein R¹ is an hydrogen and R² is a tert.butyl.

The afore mentioned compounds of formula I are described in the patent publication DE 195 40 952 or in the corresponding international publication WO 9717054 as to be UV-A light screening agents and thus do not influence the freedom of choice of the compounds effective in absorbing ultraviolet radiation in the erythermic region of 290-320 nm, i.e. UV-B. Hence, the photostabilizer compounds of formula I are particularly suitable for use in cosmetic light-screening compositions whereby the artisan of skill in this art has full freedom of choice with respect to the UV-B filter agent(s) which may be (and which are usually) incorporated in a formulation of such a composition.

These UV-B-fllters are conveniently selected according to the desired chemical and physical properties of the formulation, e.g. according to the desired degree of protection, to wavelength, εₘₐₓ, solubility, photostability, safety, see e.g. SÖFW (Journal) 122, 8 (1996) 543 seq., Cosmetics & Toiletries 107 (1992) 45 seq.

Examples of UV-B filter agents which may be incorporated are all known ones, e.g. as inter alia described in US patent No. 4,387,089 or in the patent publication DE 195 40 952 mentioned above. Preferably, the UV-B filter agent(s) is/are at least one of the group consisting of cinnamates, salicylates, benzophenones, diphenylacrylates, camphor derivates, polymeric materials and microfine pigments having a particle size in the nano- and/or low micrometer region. More preferably the UV-B filter agent(s) is/are at least one of the group consisting of metallic oxides of cerium, iron, titanium, zinc or zirconium, especially of titanium or zinc, and polymers with hydrocarbon structure or siloxane structure, i.e. polysiloxanes, carrying at least one ultraviolet-light-absorbing group.

Suitable polysiloxanes are disclosed in the above mentioned EP-A-754445 or in WO 92/20690.

Though the compounds of formula I are themselves effective in absorbing the UV-A radiation their present function is to photostabilize the above captioned UV-A-filters.

As far as the UV-A light screening agents of the novel stabilized combinations are concerned, the preferred stabilized compound is 4-tert. butyl-4'-methoxy-dibenzoylmethane (Parsol 1789®), which is disclosed, e.g. in US patent No. 4 387 089 or CH patent No. 642 536.

Other suitable compounds of the dibenzoylmethane type are: 2-methyldibenzoylmethane, 4-methyl-dibenzoyl-methane, 4-tert-butyldibenzoylmethane, 2,4-dimethyldibenzoylmethane, 2,5-dimethyldibenzoylmethane, 4,4'-diisopropyldibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane and 2,6-dimethyl-4-tert-butyl-4'-methodydibenzoylmethane.

The function of the compounds of formula I is, as pointed out above, to photostabilize the involved UV-A screening agents of above mentioned type, i.e. to guarantee a constant protection during prolonged exposure to the UV light. This way, if a repeated application of the cosmetic formulation at various intervals is required, these intervals can be extended.

The present invention thus relates to photostable dibenzoylmethane type UV-A screening agents stabilized by at least one compound of formula I 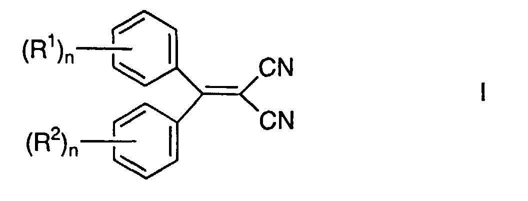
wherein R¹ and R² are equal or different and represent linear or branched alkyl or alkoxy radicals with 1 to 18 C atoms or one of R¹ and R² is a hydrogen atom and n is 1 or 2.

Specifically, R¹ and R² are alkoxy radicals with 3 to 12 C atoms or one of R¹ and R² may be a hydrogen atom whereby R¹ and/or R² are/is in para configuration and n is 1.

The alkoxy radical may especially be one of the group consisting of
n-propoxy-,
isopropoxy-,
n-butoxy-,
1-methylpropoxy-,
2-methylpropoxy-,
n-pentoxy-,
1,1-dimethylpropoxy-,
3-methylbutoxy-,
hexoxy-,
2,2-dimethylpropoxy-,
heptoxy-,
1-methyl-1-ethylpropoxy-
2-ethylhexoxy- and octoxy-.

The alkyl radical may be one of the group consisting of propyl, isopropyl, n-butyl, tert.butyl, pentyl, hexyl, preferably tert.butyl.

The dibenzoylmethane type UV-A screening agent may be one of those compounds mentioned above, preferably 4-tert.butyl-4'-dimethoxybenzoylmethane (solid under the tradename Parsol 1789®).

The photostabilizing effect was measured by the hereafter described method. Compounds of formula I wherein R¹ is a hydrogen atom and R² is H₉C₄O- or wherein R¹ and R² are both 2-ethylhexoxy or wherein R¹ is hydrogen and R² is a tert.butyl were found to be very efficient in photostabilizing a dibenzoylmethane type screening agent of UV-A type, especially in photostabilizing 4-tert.butyl-4'-methoxybenzoylmethane.

Further, the present invention relates to a cosmetic light-screening composition comprising in a cosmetically acceptable carrier containing at least one fatty phase, a photostable dibenzoylmethane type UV-A screening agent photostabilized as described above, and, optionally, at least one UV-B filter agent.

Preferably the cosmetic light-screening composition contains the photostable dibenzoylmethane UV-A type screening agent 4-tert.butyl-4'-methoxydibenzoylmethane.

The UV-B filter agent(s) of the cosmetic light-screening composition may at least be one of the group consisting of cinnamates, salicylates, benzophenones, diphenylacrylates, camphor derivates, polymeric materials and microfine pigments as mentioned above.

The UV-B filter agent(s) may preferably be at least one of the group consisting of nanopigment metallic oxides of cerium, iron, titanium, zinc or zirconium, especially of titanium or zinc, and polymers with hydrocarbon structure or siloxane structure carrying at least one ultraviolet-light-absorbing group.

The cosmetic light-screening composition comprises advantageously 0.1 to 5% by weight, preferably 0.5 to 2% by weight of the photostabilizing compound of formula I, especially of the compound of formula I wherein R¹ is a hydrogen atom and R² is H₉C₄O- or tert.butyl; or wherein R¹ and R² are both 2-ethylhexoxy.

The cosmetic light screening composition in useful for protecting human skin or human hair against ultraviolet radiation.

The compound of formula I as defined above is specifically useful as stabilizer for dibenzoylmethane type UV-A screening agents, especially of 4-tert.butyl-4'-methoxydibenzoylmethane.

The desired stabilization of the material of UV-A filters is easily established by strictly parallel experiments with the respective UV-A filters and the compounds of formula I using an appropriately equipped Xenon lamp as a solar simulator. The method is described in International Journal of Cosmetic Science 18, 167-177 (1996). Irradiated are standard preparations of the investigated products, e.g. solutions in, preferably, higher boiling cosmetic solvents, e.g. deltyl (isopropyl myristate), the resulting sunscreen being spread on glass plates. After the irradiation, the plates are immersed into a suitable solvent (e.g. ethanol) and analysed by HPLC. The stabilising effect is directly correlated to the difference in area before and after the irradiation. Usually, a combination of UV-A filter and stabiliser as exemplified below is used for the assessment.

Both components of the present combination, i.e. of the light-screening agent(s) and the photostabilizing compound of formula I, are lipophilic. The cosmetic formulations contain thus at least one fatty phase, and the formulations can consequently present themselves in the form of emulsions, lotions or gels.

Suitably the cosmetic screening composition takes the form of an oil, a lotion, a gel, a solid stick, an emulsion, e.g. cream, milk or of a vesicular dispersion of ionic or nonionic amphiphilic lipids, an aerosol, a spray, a foam, a powder, a shampoo, a hair conditioner or lacquer or a make-up or the like.

In case a cosmetic composition for the protection of human hair is prepared with the afore described photostabilized dibenzoylmethane type UV-A screening agent photostabilized by at least one compound of formula I the suitable formulations are shampoos, conditioners, lotions, gels, emulsions, dispersions, lacquers or the like. The preparation of all these formulations is well known to the skilled artisan.

The usual solvents known to the skilled practitioner can be used for the preparation of these forms, e.g. oils, waxes, alcohols, polyols. The preferred agents are fatty acids, esters, fatty alcohols, but also ethanol, isopropanol, propylene glycol, glycerine or the like are useful.

The cosmetic formulations may contain further adjuvants, e.g. further solvents, thickeners, emollients, emulsifiers, humectants, tensides, preservatives, antifoams, fragrances, oils, waxes, lower polyols and monohydric alcohols, propellants, silicones, colourings and pigments or the like.

The most important advantage of the novel photostabilizer stems from the fact that the artisan skilled in the art is completely free in the choice regarding the material used for the filtration of the UV-B radiation as already said above.

Compounds of formula I are prepared by process analogous to those known from the literature (G. Charles, Bull. Soc. Chim. Fr., 1559 (1962); P. L. Pickard and T. L. Tolbert, J. Org. Chem. 26, 4886 (1961)).

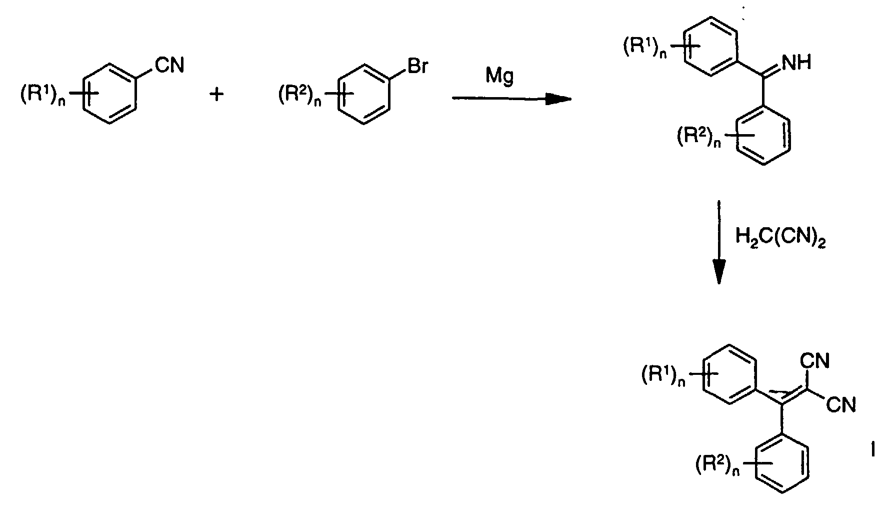

The following examples explain the invention in more detail.

### Example 1 (General procedure)

### Ketimine preparation

A Grignard-nitrile complex was prepared by the dropwise addition of 40 mmoles of nitrile to a stirred Grignard reagent prepared from 45 mmoles of halide and 46 mmoles of magnesium turning in 25 ml of anhydrous ether, followed by 4 hrs reflux. After cooling to room temperature, the stirred complex was decomposed by the dropwise addition of 270 mmoles of anhydrous methanol. The suspension was filtered and the filtrate was concentrated to give the desired ketimine.

### Compounds of formula I

Compounds of formula I were prepared by mixing 40 mmoles of the adequate ketimine with 40 mmoles of malonitrile at room temperature. Compounds of formula I were purified by chromatography. They are listed in table 1.

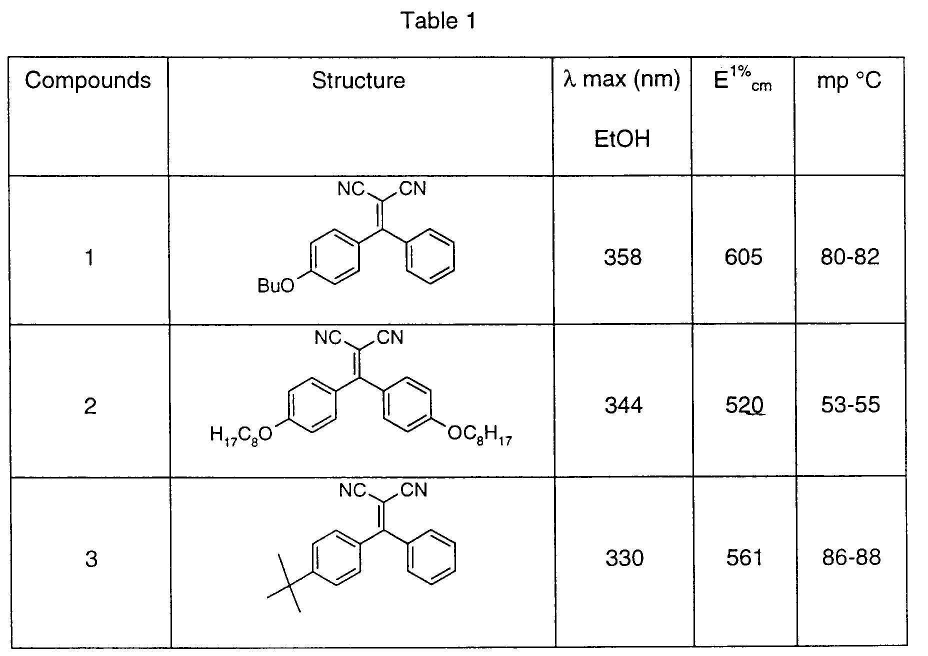

### Example 2

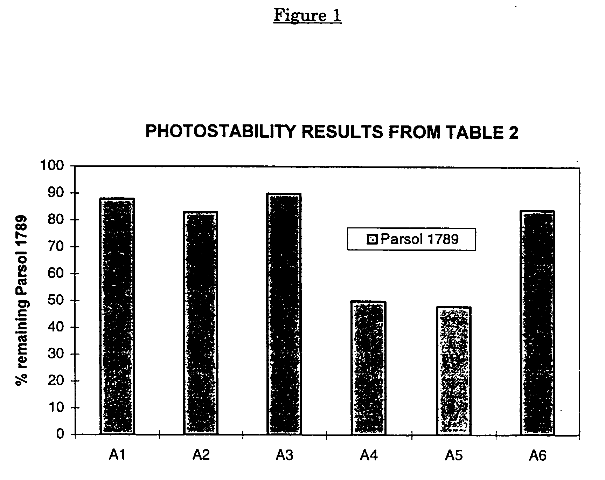

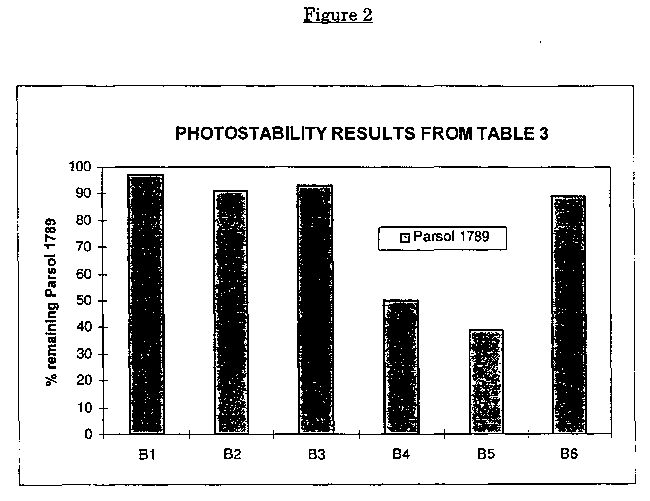

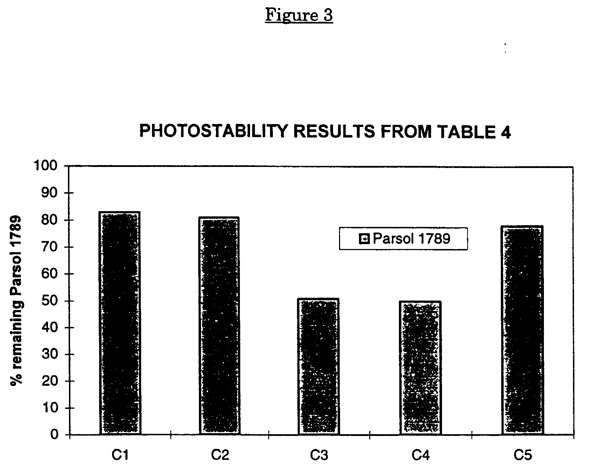

In this example, photostability experiments according to the protocol described in International Journal of Cosmetic Science 18, 167-177(1996) were performed.
The desired stabilisation of the material of UV-A filters is easily established by strict paralell experiments with the respective UV-A filter and the stabiliser using an appropriatly equipped Xenon lamp as a solar simulator. Irradiated are standard preparations of the investigated products, e.g. solutions in, preferably, higher boiling cosmetic solvents, e.g. deltyl (isopropyl myristate), the resulting solution being spread on glass plates. After irradiation, the plates are immersed into a suitable solvent (e.g. in ethanol) and analysed by HPLC. The stabilising effect is directly correlated to the difference in area before and after irradiation.
The following table shows the stabilising effect expressed as percentage with respect to non exposed sample.
Mixtures containing Parsol 1789 and compounds of formula I were irradiated in parallel with 3 controls (Parsol 1789 without stabilizer; Parsol 1789 with Benzophenone-4 (a UV-A filter); Parsol 1789 with octocrylene (a known UV-B stabilizer), for comparaison purposes. Table 2 and Fig. 1 show the results obtained using 1% of compounds according to the invention.
Table 3 and Fig. 2 show the results obtained using 2% of compounds according to the invention.

**Table 2**

| Entry | Composition (% in weight) | Remaining amount in % after irradiation |
|---|---|---|
| A1 (invention) | 2% Parsol 1789 | 88% |
| | 1% Compound 1 from table 1 | 97% |
| A2 (invention) | 2% Parsol 1789 | 83% |
| | 1% Compound 2 from table 1 | 100% |
| A3 (invention) | 2% Parsol 1789 | 90% |
| | 1% Compound 3 from table 1 | 99% |
| A4 (comparative) | 2% Parsol 1789 | 50% |
| | --- | --- |
| A5 (comparative) | 2% Parsol 1789 | 48% |
| | 1% Benzophenone-4 | 97% |
| A6 (comparative) | 2% Parsol 1789 | 84% |
| | 1% octocrylene | 100% |

**Table 3**

| Entry | Composition (% in weight) | Remaining amount in % after irradiation |
|---|---|---|
| B1 (invention) | 2% Parsol 1789 | 97% |
| | 2% Compound 1 from table 1 | 100% |
| B2 (invention) | 2% Parsol 1789 | 91% |
| | 2% Compound 2 from table 1 | 99% |
| B3 (invention) | 2% Parsol 1789 | 93% |
| | 2% Compound 3 from table 1 | 98% |
| B4 (comparative) | 2% Parsol 1789 | 50% |
| | --- | --- |
| B5 (comparative) | 2% Parsol 1789 | 39% |
| | 2% Benzophenone-4 | 94% |
| B6 (comparative) | 2% Parsol 1789 | 89% |
| | 2% octocrylene | 100% |

These results clearly show the remarquable photostabilisation effect of Parsol 1789 brought by the compounds of formula I (invention) (A1-A3 compared to A4 and B1-B3 compared to B4). These results are as good as those obtained with octocrylene (a known UV-B photostabiliser see EP-A-780119) while UV-A filter like Benzophenone-4 was inefficient.

### Example 3

Photostability experiments of emulsions containing Parsol 1789, compounds of formula I, Octocrylene and Benzophenone-4 were performed.

**Table 4**

| Oil in water emulsion | | | | | | |
|---|---|---|---|---|---|---|
| | Ingredients % w/w | C1 | C2 | C3 | C4 | C5 |
| A | Butyl Methoxydibenzoylmethane (Parsol 1789) | 2 | 2 | 2 | 2 | 2 |
| | compound 1 from table 1 | 1 | | | | |
| | compound 2 from table 1 | | 1.5 | | | |
| | Benzophenone-4 | | | | 3 | |
| | Octocrylene | | | | | 1 |
| | Glyceryl mono myristate | 4 | 4 | 4 | 4 | 4 |
| | PVP-Eicosen Copolymer | 2 | 2 | 2 | 2 | 2 |
| | Cetylalcohol | 2 | 2 | 2 | 2 | 2 |
| | Caprilic capric triglyceride | 10 | 10 | 10 | 10 | 10 |
| | Butylhydroxytoluene | 0.1 | 0.1 | 0.1 | 0.1 | 0.1 |
| | Phenoxyethanol & Methylparaben & Ethylparaben & Propylparaben & Butylparaben | 0.6 | 0.6 | 0.6 | 0.6 | 0.6 |
| | Amphisol K | 2 | 2 | 2 | 2 | 2 |
| B | Propylene Glycol | 10 | 10 | 10 | 10 | 10 |
| | Disodium EDTA | 0.1 | 0.1 | 0.1 | 0.1 | 0.1 |
| | Carbomer 981 | 10 | 10 | 10 | 10 | 10 |
| | Demineralised water qsp | 100 | 100 | 100 | 100 | 100 |

Mix part A and B separately at 85°C. Combine A and B under stirring. Finally, correct pH to 7 with potassium hydroxide 10%.

## Claims

1. Photostabilized dibenzoylmethane type UV-A screening agent stabilized by at least one compound of formula I 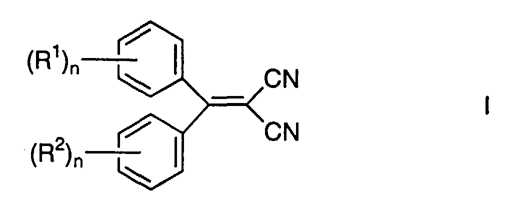
wherein R¹ and R² are equal or different and represent linear or branched alkyl or alkoxy radicals with 1 to 18 C atoms or one of R¹ and R² is a hydrogen atom and n is 1 or 2.

2. Photostabilized dibenzoylmethane type UV-A screening agent of claim 1 wherein R¹ and R² are alkoxy radicals with 3 to 12 C atoms or one of R¹ and R² is a hydrogen atom whereby R¹ and/or R² are/is in para-configuration and n is 1.

3. Photostabilized dibenzoylmethane type UV-A screening agent of claim 1 or 2 wherein the alkoxy radical is one of the group consisting of n-propoxy-, isopropoxy-, n-butoxy-, 1-methylpropoxy-, 2-methylpropoxy-, n-pentoxy-, 1,1-dimethylpropoxy-, 3-methylbutoxy-, hexyoxy-, 2,2-dimethylpropoxy-, heptoxy-, 1-methyl-1-ethylpropoxy-, 2-ethylhexoxy- and octoxy-.

4. Photostabilized dibenzoylmethane type UV-A screening agent according to claim 1 or 2 wherein R¹ is a hydrogen atom, R² is n-butoxy or tert.butyl and n is 1.

5. Photostabilized dibenzoylmethane type UV-A screening agent according to any one of claims 1-3 wherein R¹ and R² are 2-ethylhexoxy and n is 1.

6. Photostabilized dibenzoylmethane type UV-A screening agent according to any one of the claims 1 to 5 wherein the dibenzoylmethane type UV-A screening agent is one of the group consisting of 2-methyldibenzoyl-methane, 4-methyl-dibenzoyl-methane, 4-tert-butyldibenzoyl-methane, 2,4-dimethyldibenzoylmethane, 2,5-dimethyldibenzoylmethane, 4,4'-diisopropyl-dibenzoylmethane, 4-tert-butyl-4'-methodydibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane and 2,6-dimethyl-4-tert-butyl-4'-methodydibenzoylmethane.

7. Photostabilized dibenzoylmethane type UV-A screening agent according to any one of the claims 1 to 6 wherein the dibenzoylmethane screening agent is 4-tert-butyl-4'-methoxydibenzoylmethane.

8. Cosmetic light-screening composition comprising in a cosmetically acceptable carrier containing at least one fatty phase, a photostabilized dibenzoylmethane type UV-A screening agent according to any one of the claims 1 to 7, and, optionally, at least one UV-B filter agent.

9. Cosmetic light-screening composition according to claim 8 therein the UV-B filter agent is at least one of the group consisting of cinnamates, salicylates, benzophenones, diphenylacrylates, camphor derivates, polymeric materials and microfine pigments.

10. Cosmetic light-screening composition according to claim 8 or 9, wherein the UV-B filter agent is at least one of the group consisting of pigment metallic oxides of cerium, iron, titanium, zinc or zirconium, especially of titanium or zinc, and polymers with hydrocarbon structure or siloxane structure carrying at least one ultraviolet-light-absorbing group.

11. Cosmetic light-screening composition of any one of the claims 8 to 10 comprising 0,1 to 5 % by weight, preferably 0,5 to 2 % by weight, of the photostabilizing compound of formula I, especially of the compound of formula I wherein R¹ is a hydrogen atom and R² is n-butoxy or tert.butyl; or wherein R¹ and R² are 2-ethylhexoxy; and n is 1.

12. Use of a cosmetic light screening composition of any one of the claims 8 to 11 for protecting human skin or human hair against ultraviolet radiation.

13. Use of compounds of formula I as defined in any one of the claims 1 to 5 as photostabilizer for dibenzoylmethane type UV-A screening agents, especially for 4-tert-butyl-4'-methoxydibenzoylmethane-UV-A screening agent.
